# EUROPEAN PATENT APPLICATION

(11) **EP 0 756 846 A1**
(43) Date of publication of application: **05.02.1997**
(21) Application number: 96108823.4
(22) Date of filing: 03.06.1996
(51) Int. Cl.: A61B 5/00, A61B 5/0482

(54) **Electronic circuit for detecting psychokinetic effects**

(30) Priority: 05.06.1995 IT PC950013
(71) Applicant: Muti, Luciano, 24100 Bergamo (IT); Giroldini, Villiam, S. Donato Milanese (IT)
(72) Inventor: Muti, Luciano, 24100 Bergamo (IT); Giroldini, Villiam, S. Donato Milanese (IT)
(74) Representative: La Ciura, Salvatore

(57) **Abstract**

An electronic circuit (4) for detecting psychokinetic effects which is able to generate signals under the influence of mental waves, which includes a plurality of electronic amplifiers connected in series, means to detect and to amplify the variations at the output of these amplifiers caused by the influence of the mental waves, and means which transform these variations into signals suitable to be transmitted to a computer to be processed. The sum of the background noise of these amplifiers tends to have statistically a zero value, in order to distinguish the variations caused by the psychokinetic effects.

## Description

This invention relates to an electronic sensor of psychokinetic effects, that's able to produce signals under the influence of the cerebral waves, including a plurality of electronic amplifiers which are connected so that their outputs are added, a circuit for filtering and amplifying the obtained signal, and circuit means to transform these variations in signals to be sent to a computer to be worked out.

In conformity with the invention these electronic amplifiers are between 16 and 256 in number, so that the sum of the random signals is statistically null value and it's so possible to discriminate better the variations caused by the influence of the cerebral waves.

In the last 20 years numerous scientific researches, carried out by Universities in the USA and in Europe have demonstrated the human mind possibility to be able to influence directly, through the cerebral waves, different physical and biological systems.

This effect has been called psychokinetic, shortened with the abbreviation PK.

Although the entity of this PK effect is generally weak and very variable from person to person, interesting possibilities arise of application of these new knowledges in various branches of medicine, of scientific research, in the industry and in the entertainment.

In these three last applications, the revelation techniques of the PK effects are generally based on generators of random numbers (electronic circuits that produce random sequences of *bit* 0/1) or random signals that can be turned to random numbers.

These equipments, under the action of cerebral waves, can modify their exit in order to obtain a significant variation of the average ratio between bit 0 and 1, with respect to the ratio which is obtained in the absence of a mental action on the device.

These variations are generally very small and are between 0,1% and 0,4%.

To enter into details, number generators or random signals generators are based on a diode or transistor, that is used as an electronic noise source, the signal of which is amplified and also conveniently digitised to obtain a random sequence of bit 0/1.

Examples of this type are described, for example, in:
1) John R., Dunne B. Nelson R. "Engineering Anomalies research", Journal of Scientific Exploration, 1, 21-50 (1987)
2) Schmidt H. " A PK test with electronic equipment", Journal of Parapsychology, 34, 175-181 (1970)
3) Schmidt H. "Correlations between Mental Processes and external Random Events", Journal of Scientific Exploration, 4, 233-241 (1990).
   Another example of a circuit used to disclose PK effects is based on the technique of mixing the signals from a certain number of independent electronic oscillators, such as it is described in the Italian patent application N° MI 91A 000 3445 (1991).

Although it isn't known with precision the mechanism of action of the PK effect on the electronic equipments on other physical system, one of the most believable hypothesis suggests that such effects derive from little modifications of the wave function of the electrons, as some interpretation of the Quantic Mechanics assert, in which it is particularly underlined the role of the observator in the determination of the visible physical reality. (Observational Theories).

The conduction electrons of the semiconductors are typically subjected to casual fluctuations of the speed and of movement direction inside the same semiconductors (silicon, germanium, arseniur of gallium, and so on).

These quantitative fluctuations in the semiconductors are responsible for the "electronic noise", that is present in any electronic amplifier device.

As the casual fluctuations, caused by the PK effects on the conduction electrons, are generally very weak, weaker than the average electronic noise of the semiconductors, it would be extremely important to find a standard to increase the ratio signal/noise and to remarkably improve the efficiency of the sensors of PK effects.

Now it has been found a new type of generator of casual signals that is particularly suitable for the sensing of PK effects, because it has a higher sensibility, to distinguish a casual fluctuation of signal in comparison with a real psychokinetic effect.

This purpose is achieved with a circuit in accordance to the characterising part of the appended claims.

This invention will be now described in details, by way of example not restrictive, with reference to the enclosed drawings.
- Fig. 1 is the diagram of the electronic circuit in a sensor of psychokinetic effects in accordance to the invention;
- Fig. 2 shows, in outline, an equipment for the control of a videogame, including a circuit in accordance to the invention.

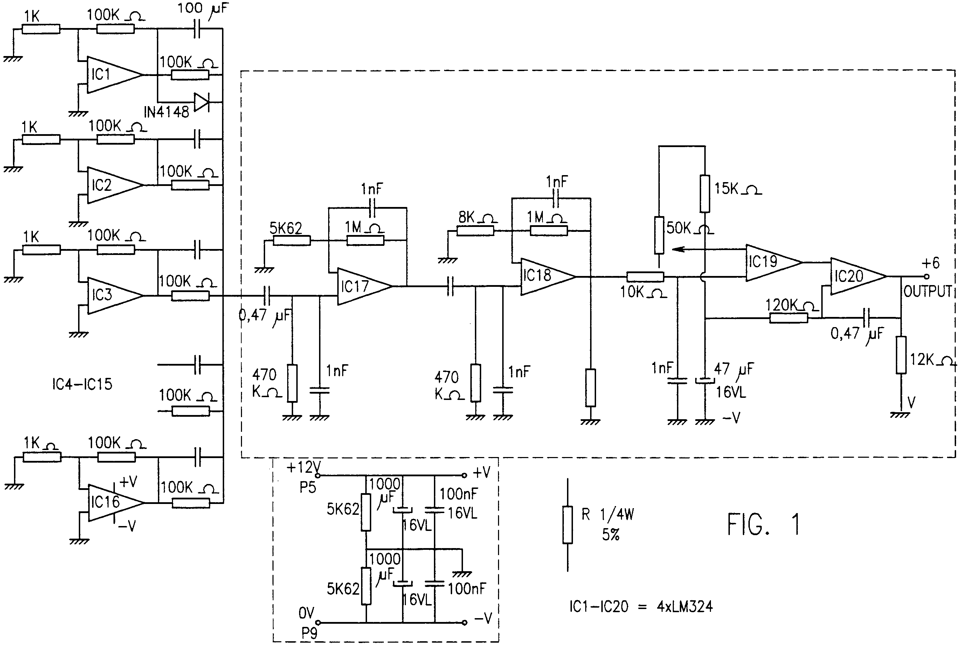

The circuit of this invention (fig. 1) performs the summation of the signals from N electronic amplifiers, N being a number between 16 and 256, working in the frequency range between 1 Hz and 10 Mhz.

Every electronic amplifier generates a signal, which is represented only by its own electronic noise, which for the operational amplifiers is typically between 0,5 microvolts and 4 microvolts.

The obtained sum-signal can also be amplified further on in order to have at the output a casual signal between 100 millivolts and 2000 millivolts and it can be filtered if necessary to limit the pass bandwidth of the signal within a range of frequencies, which is more narrow than the original one of the amplifiers.

For example, in conformity with the specific applications, it can be useful to restrict the bandwidth within a factor 100, for example between 1-100 Hz, or 10-1000 Hz or 100khz - 10Mhz.

The advantage to sum the signals of N amplifiers consists in that while the electronic noise of each amplifiers is independent from on amplifier to another , the signal caused by a weak PK effect is supposed to be identical (or almost identical) in all the amplifiers when it appears.

Under these circumstances, the sum of N casual signals tends to be statistically null, while the PK signal is added in phase with itself and therefore it isn't cancelled.

With more precision, if the electronic noise of a single amplifier is V1 µV and the PK signal, in a certain moment, has a width of V2 µV, the signal/noise ratio is s/n = V2/V1.

If the same PK signal is present at the same time inside a number N of amplifiers, then the signal/noise ratio at the output of the circuit becomes: s/n = (V2/V1)*SQR (N), where SQR (N) is the square root of the number N of amplifiers.

Therefore the signal/noise ratio improves in proportion with the square root of the number of the amplifiers, allowing to reveal in a more efficient way the weak PK effects.

The sum signal, amplified and filtered, is then straightened at full wave by a precision rectifier and at the end it is squared by a trigger circuit, the intervention threshold of which can be adjusted in order to obtain at the output, a square wave impulse succession with a variable bit ratio 0/1.

We would like to specify that "bit 0" indicates a signal with an amplitude equal or minus than 0,5 volt, while "bit 1" indicates a signal with an amplitude more than 2 volts, until 5 volts.

The adjustment of the trigger threshold can be made so as to obtain a bit 0/1 ratio between 0,1 and 10, preferably a ratio between 0,5 and 2.

The average frequency of the triggered signals is the frequency defined before as a bandwidth with a factor 100 of frequencies.

The used amplifiers are "operational amplifiers" based on the silicon or germanium or gallium arseniur technology and mostly are equipped with four operationals in a single integrated circuit as for instance the LM 324, TL084, TL064 types and so on, and they are characterised by a low thermal drift to minimise the variation of the signals emission rate at the variation of the temperature.

For a proper use of this circuit to reveal the PK effects it is important that the circuit is screened from the electromagnetic environmental disturbs.

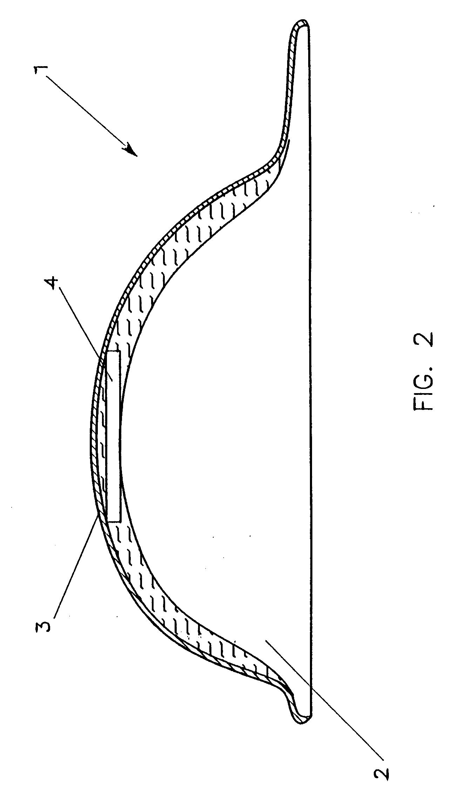

For this purpose, according to the invention the circuit (or at least the part of ciruit comprising the N amplifiers) is placed inside a screened helmet (fig. 2), in order to avoid the influence of the external magnetic fields and, suitable to be worn by the user.

For instance the helmet, indicated with number 1, comprises a plastic shell 2 coated with a layer 3 of an acrylic resin mixed with Nickel (conductor), in order to create around the head of the user an efficient screen and avoid that the circuit, schematically shown in the figures and indicated with number 4, could be disturbed by the external magnetic fields.

The circuit can be supplied through a stabilized power supply working at a voltage of 220 v, 60/60 Hz placed outside the helmet, or through batteries that can be placed inside or outside the helmet.

The digital signals from the electronic circuit may replace a common mouse or joystick and allow to interact with a computer 5 or with the subjects of a videogame with a new system based on a direct mental interaction.

The mental influence of a player can interact with the shown electronic ciruit, which transforms the weak psychokinetic effects into signal which can be used by a computer.

Because of the variable PK characteristics of any persons, the higher is the mental influence of the player, the high is the amount of electronic impulses at the output of the decoder.

These electric impulses can be used to drive a computerised informatic system through serial or parallel door, or can be transformed inside the equipment in analogic or digital way according to the foreseen kind of use.

### APPLICATIONS

### -1) The equipment can be used to reveal, to verify and to measure the psychokinetic effects.

In the most modern type such equipments are connected with a computer and relative programs, to collect the data and for their statistic analysis.

In a kind of test a subject must try to influence the decoder only my means of is own conscious will, without any direct physical contact with the equipment.

The decoder transmits the data to a computer through the parallel or serial door, and the data are processed to give a visual and or a sonorous information.

A subject placed in front of a computer watches a graphic or listen a sound, which favour the expression of the PK effect and continuously inform him about the size of the PK effect that he is obtaining (this information is named feed-back).

At the end of the test is possible to establish by statistical methods, whether a significant modification of the emission rate of bit 0/1 has taken place in comparison to the value obtained without the presence of people (control value).

### -2) Control, also only partial, of the movement of character or pictures of electronic videogames.

For this application the described generator sends impulses to the computer through the serial or parallel door and the videogame program must be built in order to use these signals to control the movement of one or more videogame characters, together with or in replace of the control by joystick or by keyboard.

In this application it is so possible to control, at least partially, the course of a videogame by a direct mental action of the player of the system.

By the adjustments of the already described trigger threshold (fig.1, potentiometer P1), it is further possible to adjust the emission rate of the random impulses 0/1 and then to modify the videogame speed, or the videogame difficulty level.

At the beginning of the videogame (set up) it could be useful to calculate the average value of the emitted electric signals, when the player is in relax, in order to have a reference value in comparison to which a difference during the course of the game can be calculated.

## Claims

1. Electronic detector psychokinetic effects able to generate signals under the influence of mental waves, characterised in that it includes a plurality of electronic amplifiers connected in series, means to detect and to amplify the variation at the output of these amplifiers caused by the influence of the mental waves, and means which transform these variations into signals suitable to be transmitted to a computer to be elaborated, so that the sum of the noise of these amplifiers tends to have statistically a zero value, in order to better distinguish the variations caused by the psychokinetic effects.

2. Psychokinetic effects electronic detector according to claim 1, characterised in that these electronic amplifiers are from 16 to 256 pieces.

3. Psychokinetic effects electronic detector according to the preceding claims, characterised in that means are provided, for rectifying and squaring the signal from said amplifiers, said means having an adjustable threshold so that to obtain at the output a square wave impulse succession with a variable bit ratio of 0/1.

4. Psychokinetic effects electronic detector according to the preceding claims, characterised in that the electronic circuit is placed inside an helmet screened against the influence of the external magnetic fields.

5. Psychokinetic effects electronic detector according to claim 4, characterised in that that the above helmet includes a plastic material shell coated with acrylic resin including conductive nickel.

6. Use of a psychokinetic effects electronic detector according to the above claim to control the movement of characters or of pictures in a videogame.
